# FASCICULE DE BREVET EUROPEEN

(11) **EP 2 646 510 B1**
(45) Date de publication et mention de la délivrance du brevet: **04.03.2015**
(21) Numéro de dépôt: 11796749.7
(22) Date de dépôt: 16.11.2011
(51) Int. Cl.: C08L 33/02, C08F 2/32, C08L 33/14, A61K 8/81, A61K 47/32, A61Q 1/00, A61Q 5/00, A61Q 17/04, A61Q 19/00

(54) **NOUVEL ÉPAISSISSANT POLYMÉRIQUE EXEMPT DE TOUT FRAGMENT ACRYLAMIDO, PROCÉDÉ POUR LEUR PRÉPARATION ET COMPOSITION EN CONTENANT**
NEUARTIGES POLYMERES VERDICKUNGSMITTEL OHNE ACRYLAMIDFRAGMENTE, VERFAHREN ZUR SEINER HERSTELLUNG UND ZUSAMMENSETZUNG DAMIT
NOVEL POLYMERIC THICKENING AGENT FREE OF ACRYLAMIDE FRAGMENTS, METHOD FOR THE PREPARATION THEREOF, AND COMPOSITION CONTAINING SAME

(30) Priorité: 30.11.2010 FR 1059944
(43) Date de publication de la demande: 09.10.2013
(73) Titulaire: Societe D'Exploitation De Produits Pour Les Industries Chimiques Seppic, 75007 Paris (FR)
(72) Inventeur: BRAUN, Olivier, 81100 Castres (FR); MALLO, Paul, 78290 Croissy-Sur-Seine (FR)
(74) Mandataire: Conan, Philippe Claude
(86) Numéro de dépôt international: PCT/FR2011/052648
(87) Numéro de publication internationale: WO 2012/072911

(56) Documents cités:
- EP-A1- 0 017 025
- DE-A1-102009 014 877

## Description

L'invention a pour objet, de nouveaux épaississants anioniques polymères leur procédé de préparation ainsi que leur utilisation comme agent épaississant et/ou émulsionnant.

L'épaississement des phases aqueuses est en général réalisé en y incorporant des polymères hydrophiles de toutes sortes, qu'ils soient synthétiques ou d'origine naturelle.

Parmi les polymères d'origine naturelle, les gommes xanthane ou de guar sont assez largement utilisées. Ils ont cependant les inconvénients classiques des produits naturels, à savoir une qualité et un prix fluctuant.

Parmi les épaississants synthétiques hydrophiles les plus largement utilisés, il y a les polymères sous forme de poudres ou de latex inverses auto-inversibles. En terme de structure chimique, il y a notamment les homopolymères de l'acide acrylique sous forme libre ou partiellement salifiée ; les homopolymères sous forme libre sont généralement synthétisés par polymérisation précipitante et se présentent sous forme de poudre ; les homopolymères sous forme partiellement salifiée sont quant à eux généralement préparés soit par polymérisation précipitante soit par polymérisation en émulsion inverse. Il y a par exemple les latex inverses auto-inversibles divulgués dans la demande brevet européen publiée sous le numéro EP 1 010 708 A1, dans lesquels l'homopolymère de l'acide acrylique est salifié sous forme de sel d'ammonium ou de sel de monoéthanol amine. Or le pouvoir épaississant des polymère de cette première famille n'est pas entièrement satisfaisant et leur mauvaise odeur due à la présence d'un base azotée, limite leur utilisation en cosmétique. La demande de brevet européen publiée sous le numéro EP 0 17 025 A1 divulgue des copolymères de l'acide acrylique avec le dimère de l'acide acrylique qui sont utilisés comme couche de film photographiques. La demande de brevet allemand publiée sous le numéro DE 10 2009 014 877 A1 divulgue des copolymères de l'acide acrylamido-2-méthyl-1-propanesulfonique et de l'acrylate de carboxyéthyle, qui sont utilisés en cosmétique.

Les inventeurs ont cherché à développer des polymères épaississants ayant un pouvoir épaississant meilleur que les polymères acryliques de l'état de la technique et n'ayant cet inconvénient en terme de mauvaise odeur.

Selon un premier aspect, l'invention a pour objet composition comprenant une phase huile, une phase aqueuse, au moins un système émulsionnant de type eau-dans huile, sous forme d'un latex inverse caractérisée en ce qu'elle comprend un polyélectrolyte anionique linéaire, branché ou réticulé, comprenant :
a) une proportion molaire non nulle, d'unités monomériques issues de l'acide acrylique sous forme libre ou partiellement salifié, et
b) une proportion molaire non nulle d'unité monomériques issues de l'acide de formule (I) :

   CH₂=CH-C(=O)-O-[CH₂-CH₂-C(=O)-O]ₙ-H (I),
dans laquelle n représente un nombre supérieur ou égal à 1 et inférieur ou égal à 5, ou d'un mélange d'acides de formule (I), sous forme libre ou partiellement salifiée.

Par latex inverse on désigne, une émulsion eau dans huile d'au moins un polymère.

Par polyélectrolyte branché, on désigne un polyélectrolyte non linéaire qui possède des chaînes pendantes de manière à obtenir, lorsqu'il est mis en solution dans l'eau, un fort état d'enchevêtrement conduisant à des viscosités, à bas gradient de vitesse, très importantes.

Par polyélectrolyte réticulé, on désigne un polyélectrolyte non linéaire se présentant à l'état de réseau tridimensionnel insoluble dans l'eau, mais gonflable à l'eau et conduisant donc à l'obtention d'un gel chimique.

Dans la définition du latex inverse tel que défini précédemment, le terme salifié indique qu'il s'agit de sels de métaux alcalins tels que les sels de sodium ou de potassium.

Le polyélectrolyte constitutif dudit latex inverse peut comporter des motifs réticulés et/ou des motifs branchés.

Dans la composition telle que définie ci-dessus, le système émulsionnant de type "eau dans huile" (E/H) est constitué soit d'un seul tensioactif soit d'un mélange de tensioactifs, à condition que ledit tensioactif ou ledit mélange ait une valeur de HLB suffisamment faible pour induire une émulsion eau dans huile. Il y a par exemple les esters de sorbitan, comme l'oléate de sorbitan, commercialisé par la société SEPPIC sous le nom MONTANE™ 80, l'isostéarate de sorbitan, commercialisé par la société SEPPIC sous le nom MONTANE™ 70 ou le sesquioléate de sorbitan commercialisé par la société SEPPIC sous le nom MONTANE™ 83. Il y aussi certains esters de sorbitan polyéthoxylés, par exemple le mono-oléate de sorbitan pentaéthoxylé commercialisé par la société SEPPIC sous le nom MONTANOX™ 81 ou l'isostéarate de sorbitan pentaéthoxylé commercialisé sous le nom MONTANOX™ 71 par la société SEPPIC. Il y a encore les polyesters de poids moléculaire compris entre 1000 et 3000, produits de la condensation entre un acide poly(isobutènyl) succinique ou son anhydride et tels que l'HYPERMER™ 2296 commercialisé par la société UNIQEMA ou enfin les copolymères blocks de poids moléculaire compris entre 2500 et 3500, comme l'HYPERMER™ B246 commercialisé par la société UNIQEMA ou le SIMALINE™ IE 200 commercialisé par la société SEPPIC.

La composition telle que définie précédemment comprend généralement entre 0,5% massique et 10% massique dudit système émulsionnant de type "eau dans huile".

Le latex inverse comprend généralement pour 100% massique, de 1% et 50% massique d'eau.

La phase huile du latex inverse auto-inversible décrit ci-dessus, est constituée :
- Soit par une huile minérale, ou par un mélange d'huile minérales, contenant des hydrocarbures saturés de type paraffine, isoparaffine, cycloparaffine, présentant à température ambiante, une densité entre 0,7 et 0,9 et un point d'ébullition supérieur à 180°C, telle que par exemple, l'ISOPAR^{™} M ou l'ISOPAR^{™} L, l'EXXOL^{™} D 100 S commercialisé par EXXON ou les huiles blanches minérales conformes aux réglementations FDA 21 CFR 172.878 et FR 178.3620(a), telles que le MARCOL^{™} 52 ou le MARCOL^{™} 82, également commercialisées par EXXON ;
- Soit par une huile de synthèse, ou par un mélange d'huile de synthèse, telle que les polyisobutènes hydrogéné, notamment ceux commercialisés en France par la société Ets B. Hossow et Cie sous le nom PARLEAM - POLYSYNLANE^{™} et cité dans Michel and Irene Ash ; Thesaurus of Chemical products, Chemise Publicité Cos, Ince. 1986 Volume I, page 211 (ISBN 0 7131 36030) ; les polydécènes ; l'isohexadécane, identifié dans Chemical Abstracts par le numéro RN = 93685 - 80 - 4 et qui est un mélange d'isoparaffines en C₁₂, C₁₆ et C₂₀ contenant au moins 97% d'isoparaffimes en C₁₆, parmi lesquelles le constituant principal est le 2,2,4,4,6,8,8-heptaméthyl nonane (RN = 4390-04-9), commercialisé en France par la société Bayer ; l'isododécane, commercialisé en France par la société Bayer ;
- Soit par une huile végétale, ou par un mélange d'huiles végétales, telle que le squalane qui est identifié dans Chemical Abstracts par le numéro RN = 111-01-3 et qui est un mélange d'hydrocarbures contenant plus de 80% massique de 2,6,10,15,19,23-hexaméthyl tétracosane, ou une huile végétale de type ester ou triglycéride, comme le coco caprylate caprate, par exemple le DUB™ 810C fourni par la société Dubois, ou encore l'huile de Jojoba ;
- Soit par un mélange de plusieurs de ces différentes huiles.

La composition telle que définie précédemment comprend généralement pour 100% massique de 5% à 50% massique d'huile.

Selon un aspect particulier le polyélectrolyte constitutif de la composition objet de la présente invention, comprend une proportion molaire non nulle d'unité monomériques issues de l'acide de formule (I₁) :

CH₂=CH-C(=O)-O-CH₂-CH₂-C(=O)-O-H (I₁),

correspondant à la formule (I) telle que définie précédemment dans laquelle n représente un nombre égal à 1, sous forme libre ou partiellement salifiée.

Selon un autre aspect particulier le polyélectrolyte constitutif de la composition objet de la présente invention, comprend une proportion molaire non nulle d'unité monomériques issues d'un mélange de l'acide de formule (I₁) :

CH₂=CH-C(=O)-O-CH₂-CH₂-C(=O)-O-H (I₁),

correspondant à la formule (I) telle que définie précédemment dans laquelle n représente un nombre égal à 1, et de l'acide de formule (I₂) :

CH₂=CH-C(=O)-O-CH₂-CH₂-C(=O)-O-CH₂-CH₂-C(=O)-OH (I₂),

correspondant à la formule (I) telle que définie précédemment dans laquelle n représente un nombre égal à 2, lesdits acides étant sous forme libre ou partiellement salifiée, dans un ratio molaire (I₁)/(I₂) supérieur à 1 et plus particulièrement supérieur à 1,5.

Selon un autre aspect particulier le polyélectrolyte constitutif de la composition objet de la présente invention, comprend une proportion molaire non nulle d'unité monomériques issues d'un mélange de l'acide de formule (I₁) :

CH₂=CH-C(=O)-O-CH₂-CH₂-C(=O)-O-H (I₁),

correspondant à la formule (I) telle que définie précédemment dans laquelle n représente un nombre égal à 1, de l'acide de formule (I₂) :

CH₂=CH-C(=O)-O-CH₂-CH₂-C(=O)-O-CH₂-CH₂-C(=O)-OH (I₂),

correspondant à la formule (I) telle que définie précédemment dans laquelle n représente un nombre égal à 2, et de l'acide de formule (I₃) :

CH₂=CH-C(=O)-O-CH₂-CH₂-C(=O)-O-CH₂-CH₂-C(=O)-O-CH₂-CH₂-C(=O)-OH (I₃),

correspondant à la formule (I) telle que définie précédemment dans laquelle n représente un nombre égal à 3, lesdits acides étant sous forme libre ou partiellement salifiée, dans un ratio molaire (I₁)/(I₂) supérieur à 1 et plus particulièrement supérieur à 1,5 et dans un ratio molaire (I₂)/(I₃) supérieur à 1,5 et plus particulièrement supérieur ou égal à 2.

Selon un aspect particulier, le polyélectrolyte anionique constitutif du latex inverse tel que défini ci-dessus, comprend pour 100% molaire :
- De 90% molaire à 99,5% molaire et plus particulièrement de 95% molaire à 99% molaire d'unités monomériques issues de l'acide acrylique libre ou partiellement salifié ;
- De 0,5% molaire à 10% molaire et plus particulièrement de 1% molaire à 5% molaire d'unités monomériques issues de l'acide carboxylique de formule (I), libre ou partiellement salifié.

Selon un autre aspect particulier de la présente invention, le polyélectrolyte anionique tel que défini précédemment est réticulé.

Dans ce dernier cas l'agent de réticulation est choisi notamment parmi les composés diéthyléniques ou polyéthyléniques, et tout particulièrement parmi l'acide diallyloxyacétique ou un des sels et notamment son sel de sodium, le triallylamine ou ses sels, le triméthylol propanetriacrylate, le diméthacrylate d'éthylèneglycol, le diacrylate de diéthylène glycol, le diallylurée ou le méthylène bis(acrylamide).

selon un aspect particulier de la présente invention l'agent réticulant mis en oeuvre est le triallylamine ou le méthylène bis(acrylamide).

L'agent de réticulation est alors généralement mis en oeuvre dans la proportion molaire exprimée par rapport aux monomères mis en oeuvre, de 0,005% molaire à 1% molaire.

Selon un autre aspect particulier de la présente invention la composition telle que définie précédemment comprend en outre un système émulsionnant de type "huile-dans eau".

Ledit "système émulsionnant du type huile dans eau " (H/E), est constitué soit d'un seul tensioactif soit d'un mélange de tensioactifs, à condition que ledit tensioactif ou ledit mélange ait une valeur de HLB suffisamment élevée pour induire une émulsion huile dans eau. Il ya par exemple :
- Les esters de sorbitan éthoxylés comme l'oléate de sorbitan polyéthoxylé avec 20 moles d'oxyde d'éthylène commercialisé par la société SEPPIC sous le nom de MONTANOX™ 80 ou le laurate de sorbitan polyéthoxylé avec 20 moles d'oxyde d'éthylène commercialisé par la société SEPPIC sous le nom de MONTANOX^{™} 20 ;
- L'huile de ricin polyéthoxylée avec 40 moles d'oxyde d'éthylène commercialisé sous le nom SIMULSOL^{™} OL50 ;
- L'alcool oléodécylique décaéthoxylé, commercialisé par la société SEPPIC sous le nom SIMULSOL^{™} OC 710 ;
- L'alcool laurique heptaéthoxylé commercialisé sous le nom SIMULSOL^{™} P7 ; ou
- Les hexaoléates de sorbitan polyéthoxylés commercialisés par la société SEPPIC sous le nom SIMALINE IE 400.

Lorsqu'elle comprend en outre un système émulsionnant de type "huile dans eau", la composition telle que définie précédemment est alors sous forme d'un latex inverse auto-inversible qui comprend généralement de 1% massique à 15% massique dudit système émulsionnant de type huile dans eau".

La composition selon l'invention peut également contenir divers additifs tels que des agents complexants ou des agents limiteurs de chaîne.

Selon un autre aspect particulier de la présente invention, celle-ci a pour objet une composition telle que définie précédemment comprenant de 15% à 60% massique, et de préférence de 25% à 40% massique, dudit polyélectrolyte anionique.

Selon un autre aspect particulier de la présente invention, celle-ci a pour objet une composition telle que définie précédemment comprenant plus de 60% jusqu'à à 80% massique, et de préférence plus de 60% à 70% massique, dudit polyélectrolyte anionique.

Le composé de formule (I) dans laquelle n est égal à 1, dénommé acide 4-oxa 5-oxo hept-6-ènoïque ou acrylate de β-carboxy éthyle, est un produit disponible dans le commerce. Il est identifié sous le numéro CAS = 24615-84-7.

Selon un autre aspect de la présente invention, celle-ci a pour objet un procédé de préparation de la composition telle que définie à l'une quelconque des précédemment, comprenant les étapes suivantes :
Une étape a) au cours de laquelle une solution aqueuse comprenant les monomères et les éventuels additifs hydrophiles, est émulsionnée dans une phase huile comprenant les monomères et les éventuels additifs lipophiles en présence dudit système émulsifiant de type eau dans huile ;
Une étape b) au cours de laquelle la réaction de polymérisation est amorcée par introduction dans l'émulsion formée à l'issue de l'étape a), d'un initiateur de radicaux libres et d'éventuellement un co-initiateur puis laissée se dérouler pour obtenir ladite composition sous forme d'un latex inverse.

Lors de l'étape b) du procédé tel que défini la réaction de polymérisation est généralement amorcée par un couple oxydoréducteur générateur d'ions hydrogénosulfite (HSO₃) tel que le couple hydroperoxyde de cumène - métabisulfite de sodium (Na₂S₂O₅) ou le couple hydroperoxyde de cumène-chlorure de thionyle (SOCl₂) à une température inférieure ou égale à 10°C, si désiré accompagné d'un agent co-initiateur de polymérisation tel que par exemple l'azo-bis(isobutyronitrile), le dilauroylperoxyde ou le persulfate de sodium puis conduite soit de manière quasi adiabatique jusqu'à une température supérieure ou égale à 50°C, soit en contrôlant la température.

Selon un aspect particulier, le procédé tel que défini ci-dessus, comprend en outre une étape c), au cours de laquelle ledit système émulsionnant de type huile dans eau est ajouté au latex-inverse formé à l'issue de l'étape b), pour obtenir ladite composition sous forme d'une latex inverse auto-inversible.

Lors de l'étape c) du procédé tel que défini ci-dessus, l'ajout dudit système émulsionnant de type huile dans eau, l'ajout se fait généralement à une température inférieure ou égale à 50°C.

Selon un aspect particulier, le procédé tel que défini ci-dessus, comprend en outre une étape b₁, au cours de laquelle le latex inverse issu de l'étape b) est concentré, pour obtenir ladite composition sous forme d'un latex inverse concentré, avant la mise en oeuvre le cas échéant, de l'étape c).

Selon un aspect particulier, le procédé tel que défini ci-dessus, comprend en outre une une étape c₁, au cours de laquelle le latex inverse auto-inversible issu de l'étape c) est concentré, pour obtenir ladite composition sous forme d'un latex inverse auto-inversible concentré.

Lors de l'étape b₁ ou de l'étape c₁ du procédé tel que défini ci-dessus, la concentration du milieu est généralement réalisé par distillation jusqu'à atteindre la teneur souhaitée en polyélectrolyte anionique au sein de la composition objet de la présente invention.

Selon un aspect particulier, le procédé tel que défini ci-dessus, comprend en outre une étape d) au cours de laquelle le latex inverse issu de l'étape b), le latex inverse concentré issu de l'étape b1), le latex inverse auto-inversible issu de l'étape c) ou le latex inverse auto-inversible concentré issu de l'étape c1), est séché par atomisation, pour former une poudre dudit polyélectrolyte anionique.

L'invention a aussi pour objet une poudre du polyélectrolyte anionique linéaire ou réticulé caractérisée en ce qu'elle est obtenue par le procédé tel que défini précédemment.

Le polyélectrolyte anionique objet de la présente invention ainsi que les latex inverses et les latex inverses auto-inversibles auto-inversibles en comportant, sont avantageusement utilisés comme épaississants et/ou comme émulsionnant/stabilisant dans des compositions cosmétiques ou pharmaceutiques.

C'est pourquoi selon un autre aspect, l'invention a pour objet, l'utilisation de la composition ou de la poudre telles que définies précédemment, comme agent épaississant et/ou comme agent émulsionnant dans des compositions cosmétiques ou pharmaceutiques.

La poudre ou composition sous forme de latex inverse éventuellement auto-inversible, objet de la présente invention peuvent être formulés dans des formules cosmétiques ou pharmaceutiques comme des mousses, des gels, des lotions, des sprays, des shampoings, des conditionneurs, des lotions pour les mains et le corps, des écrans solaires, et plus généralement dans des produits de soin.

Dans le cas du traitement ou de l'entretien du cheveu, de telles compositions cosmétiques ou pharmaceutiques se présentent habituellement sous forme de shampoings d'émulsions, de microémulsions et notamment dans le cas des conditionneurs, d'émulsions vaporisables.

Selon un dernier aspect l'invention a pour objet, une composition cosmétique ou pharmaceutique caractérisée en en ce qu'elle contient comme agent émulsionnant et/ou épaississant, une quantité efficace de la composition de la poudre telles que définies précédemment.

Par quantité efficace, on entend une proportion pondérale comprise entre environ 1% et environ 10% en poids de la composition telle que définie précédemment et environ 0,2% massique à environ 5% de la poudre telle que définie précédemment.

Les exemples suivants illustrent la présente invention sans toutefois la limiter.

### Exemple A : Latex inverse auto-inversible d'un homopolymère d'acide acrylique partiellement salifié sous forme d'un sel de sodium réticulé au triallylamine (état de la technique)

### Préparation du latex inverse auto-inversible

- On prépare une phase huile en mélangeant :
   261g de MARCOL™ 52,
   15,5 g de MONTANE™ 80™ VG, et
   3,5g de MONTANOX™ 81 : 3,5 g
- On prépare une phase aqueuse en introduisant :
   203g d'acide acrylique glacial,
   188 g d'une solution aqueuse à 48% massique d'hydroxyde de sodium,
   0,45 g de VERSENEX™ 80
   0,77 g de triallyl amine puis on complète avec de l'eau permutée jusqu'à 653 g
- On introduit la phase aqueuse dans la phase huile et on émulsifie à l'aide d'un appareil type ULTRA TURRAX™
- Après avoir laissé le système sous agitation et barbotage d'azote, on le refroidit 10°C et on ajoute y 10cm3 d'une solution à 0,268% massique d'hydroperoxyde de cumène dans le MARCOL™ 52, puis progressivement 20g d'une solution aqueuse à 0,54% massique de métabisulfite de sodium.
- On laisse la température monter dans le milieu réactionnel jusqu'à 80 °C puis laisse la réaction se dérouler pendant une heure. On introduit dans le latex inverse final obtenu, 30 g de MONTANOX™80.

### Analyse viscosimétrique du latex inverse auto-inversible obtenu

Viscosité d'une dispersion aqueuse de 3% massique du latex inverse auto-inversible : 55.800 mPas (BROOKFIELD™ RVT, axe 6, Vitesse : 5 tours par minute)

Viscosité d'une dispersion aqueuse contenant 0,1% massique de chlorure de sodium, et 3% massique du latex inverse auto-inversible :
3.200 mPas (BROOKFIELD™ RVT, axe 3, 5 tours par minutes)

### Exemple 1 : Latex inverse auto-inversible d'un copolymère acide acrylique - acrylate de β-carboxy éthyle partiellement salifié sous forme d'un sel de sodium réticulé au triallylamine dans le MARCOL™52 (Composition 1)

### Préparation du latex inverse auto-inversible

- On prépare une phase huile en mélangeant :
   261g de MARCOL™ 52,
   15,5 g de MONTANE™ 80™ VG, et
   3,5g de MONTANOX™ 81 : 3,5 g
- On prépare une phase aqueuse en introduisant :
   186g d'acide acrylique glacial,
   17,3g d'acrylate de β-carboxy éthyle commercialisé par la société BIMAX sous le nom BETA-C™
   188 g d'une solution aqueuse à 48% massique d'hydroxyde de sodium,
   0,45 g de VERSENEX™ 80
   0,77 g de triallyl amine puis on complète avec de l'eau permutée jusqu'à 653 g
- On introduit la phase aqueuse dans la phase huile et on émulsifie à l'aide d'un appareil type ULTRA TURRAX™
- Après avoir laissé le système sous agitation et barbotage d'azote, on le refroidit 10°C et on ajoute y 10cm3 d'une solution à 0,268% massique d'hydroperoxyde de cumène dans le MARCOL™ 52, puis progressivement 20g d'une solution aqueuse à 0,54% massique de métabisulfite de sodium.
- On laisse la température monter dans le milieu réactionnel jusqu'à 80 °C puis laisse la réaction se dérouler pendant une heure. On introduit dans le latex invernse final obtenu, 30 g de MONTANOX™80.

### Analyse viscosimétrique du latex inverse auto-inversible obtenu

Viscosité d'une dispersion aqueuse de 3% massique du latex inverse auto-inversible : 80.400 mPas (BROOKFIELD™ RVT, axe 6, Vitesse : 5 tours par minute)

Viscosité d'une dispersion aqueuse contenant 0,1% massique de chlorure de sodium, et 3% massique du latex inverse auto-inversible :
5.600 mPas (BROOKFIELD™ RVT, axe 3, 5 tours par minutes)

Cette analyse permet de mettre en évidence un pouvoir épaississant plus accentué que celui du latex inverse auto-inversible de l'exemple A, qui est du à la présence d'unités monomériques issu de l'acrylate de β-carboxy éthyle. Une légère amélioration de la tenue aux sels doit être aussi notée.

### Exemple 2 : Latex inverse auto-inversible d'un copolymère acide acrylique - acrylate de β-carboxy éthyle partiellement salifié sous forme d'un sel de sodium réticulé au triallylamine dans le squalane (Composition 2)

On procède comme à l'exemple 1 en substituant le MARCOL™52 par du SQUALANE™ VG. On obtient le latex inverse auto-inversible attendu.

### Exemple 3 : Latex inverse auto-inversible d'un copolymère acide acrylique - acrvlate de β-carboxy éthyle partiellement salifié sous forme d'un sel de sodium réticulé au triallylamine dans l'ISOPAR™M (Composition 3)

On procède comme à l'exemple 1 en substituant le MARCOL™52 par l'ISOPAR™M. On obtient le latex inverse auto-inversible attendu.

### Exemple 4 : Poudre de copolymère acide acrylique - acrylate de β-carboxy éthyle partiellement salifié sous forme d'un sel de sodium réticulé au triallylamine

Le latex inverse auto-inversible obtenu à l'exemple précédent est concentré par distillation, puis séché par atomisation.

### Exemples de formulations.

### Exemple 5 : Crème de soin

| | |
|---|---|
| Cyclométhicone : | 10% |
| Composition 1 : | 4% |
| MONTANOV™ 68 : | 4,50% |
| Conservateur : | 0,65% |
| Lysine : | 0,025% |
| EDTA (sel disodique) : | 0,05% |
| Gomme de xanthane : | 0,20% |
| Glycérine : | 3% |
| Eau : | qsp 100% |

### Exemple 6 : Crème de soin

| | |
|---|---|
| Cyclométhicone : | 10% |
| Composition 2 : | 4% |
| MONTANOV™ 68 : | 4,5% |
| Perfluoropolymethylisopropylether : | 0,5% |
| Conservateur : | 0,65% |
| Lysine : | 0,025% |
| EDTA (sel disodique) : | 0,05% |
| PEMULEN™ TR : | 0,2% |
| Glycérine : | 3% |
| Eau : | qsp 100% |

### Exemple 7 : Baume après-rasage

| FORMULE | | |
|---|---|---|
| A | Composition 3 : | 3% |
| | Eau : | q.s.p 100% |
| B | MICROPEARL™ M 100: | 5,0% |
| | SEPICIDE™ CI: | 0,50% |
| | Parfum: | 0,20% |
| | Ethanol 95°: | 10,0% |
| MODE OPERATOIRE : Ajouter B dans A. | | |

### Exemple 8 : Emulsion satinée pour le corps

| FORMULE | | |
|---|---|---|
| A | SIMULSOL™ 165 : | 5,0% |
| | LANOL™ 1688 : | 8,50% |
| | Beurre de Karité : | 2% |
| | Huile de paraffine : | 6,5% |
| | LANOL™ 14M : | 3% |
| | LANOL™ S : | 0,6% |
| B | Eau : | 66,2% |
| C | MICROPEARL™ M 100: | 5% |
| D | Poudre de l'exemple 4 : | 1% |
| E | SEPICIDE™ CI : | 0,3% |
| | SEPICIDE™ HB : | 0,5% |
| | MONTEINE™ CA : | 1% |
| | Parfum : | 0,20% |
| | Acétate de vitamine E : | 0,20% |
| | Sodium pyrolidinone carboxylate: | 1% (agent hydratant) |

MODE OPERATOIRE : Ajouter C dans B, émulsionner B dans A à 70°C, puis ajouter D à 60°C puis E à 30°C.

### Exemple 9 : Lait corporel

| FORMULE | | |
|---|---|---|
| A | SIMULSOL™ 165: | 5,0% |
| | LANOL™ 1688: | 12,0% |
| | LANOL™ 14M: | 2,0% |
| | Alcool cétylique: | 0,3% |
| | SCHERCEMOL™ OP : | 3% |
| B | Eau : | q.s.p. 100% |
| C | Composition 1 : | 0,35% |
| D | SEPICIDE™ CI: | 0,2% |
| | SEPICIDE™ HB : | 0,5% |
| | Parfum: | 0,20% |

MODE OPERATOIRE : Emulsionner B dans A vers 75°C ; ajouter C vers 60°C, puis D vers 30°C.

### Exemple 10 : crème H/E

| FORMULE | | |
|---|---|---|
| A | SIMULSOL™ 65: | 5,0% |
| | LANOL™ 1688: | 20,0% |
| | LANOL™ P: | 1,0% (additif à effet stabilisant) |
| B | Eau : | q.s.p. 100% |
| C | Composition 1 : | 2,50% |
| D | SEPICIDE™ CI : | 0,20% |
| | SEPICIDE™ HB : | 0,30% |

MODE OPERATOIRE : Introduire B dans A vers 75°C ; ajouter C vers 60°C, puis D vers 45°C.

### Exemple 11 : gel solaire non gras

| FORMULE | | |
|---|---|---|
| A | Composition 3 : | 3,00% |
| | Eau : | 30% |
| B | SEPICIDE™ C : | 0,20% |
| | SEPICIDE™ HB : | 0,30% |
| | Parfum : | 0,10% |
| C | Colorant : | q.s. |
| | Eau : | 30% |
| D | MICROPEARL™ M 100 : | 3,00% |
| | Eau : | q.s.p 100% |
| E | Huile de silicone : | 2,0% |
| | PARSOL™ MCX : | 5,00% |

MODE OPERATOIRE : Introduire B dans A; ajouter C puis D puis E.

### Exemple 12 : Lait solaire

| FORMULE | | |
|---|---|---|
| A | SEPIPERL™ N : | 3,0% |
| | Huile de sésame : | 5,0% |
| | PARSOL™ MCX : | 5,0% |
| | Carraghénane λ : | 0,10% |
| B | Eau : | q.s.p.100% |
| C | Poudre de l'exemple 4 : | 0,80% |
| D | Parfum: | q.s. |
| | Conservateur : | q.s. |

MODE OPERATOIRE : Emulsionner B dans A à 75°C puis ajouter C vers 60°C, puis D vers 30°C et ajuster le pH si nécessaire.

### Exemple 13 : Gel de massage

| FORMULE | | |
|---|---|---|
| A | Composition 3 : | 3,5% |
| | Eau : | 20,0% |
| B | Colorant : | 2 gouttes/100g |
| | Eau : | q.s. |
| C | Alcool : | 10% |
| | Menthol : | 0,10% |
| D | Huile de silicone : | 5,0% |
| MODE OPERATOIRE : Ajouter B dans A; puis ajouter au mélange, C puis D. | | |

### Exemple 14 : gel soin de massage

| FORMULE | | |
|---|---|---|
| A | Composition 1 : | 3,00% |
| | Eau : | 30% |
| B | SEPICIDE™ CI: | 0,20% |
| | SEPICIDE™ HB: | 0,30% |
| | Parfum: | 0,05% |
| C | colorant : | q.s. |
| | Eau : | q.s.p 100% |
| D | MICROPEARL™ SQL: | 5,0% |
| | LANOL™ 1688 : | 2% |
| MODE OPERATOIRE : Préparer A; additionner B, puis C, puis D. | | |

**Exemple 15 : Gel coup d'éclat**

| FORMULE | | |
|---|---|---|
| A | Composition 2 : | 4% |
| | Eau : | 30% |
| B | ELASTINE™ HPM: | 5,0% |
| C | MICROPEARL™ M 100: | 3% |
| | Eau : | 5% |
| D | SEPICIDE™ CI : | 0,2% |
| | SEPICIDE™ HB : | 0,3% |
| | Parfum: | 0,06% |
| | Sodium pyrolidinone carboxylate 50% : | 1% |
| | Eau : q.s.p. | 100% |

MODE OPERATOIRE : Préparer A; additionner B, puis C, puis D.

### Exemple 16 : Lait corporel

| FORMULE | | |
|---|---|---|
| A | SEPIPERL™ N : | 3,0% |
| | Triheptonate de glycerol : | 10,0% |
| B | Eau : | q.s.p.100% |
| C | Poudre de l'exemple 4 : | 1,0% |
| D | Parfum: | q.s. |
| | Conservateur : | q.s. |
| MODE OPERATOIRE : Fondre A à environ 75°C. Emulsionner B dans A à 75°C puis ajouter C vers 60°C, puis D. | | |

### Exemple 17 : Emulsion démaquillante à l'huile d'amande douce

| FORMULE | |
|---|---|
| MONTANOV™ 68 : | 5% |
| Huile d'amandes douces : | 5% |
| Eau : | q.s.p.100% |
| Poudre de l'exemple 4 : | 0,3% |
| Glycérine : | 5% |
| Conservateur : | 0,2% |
| Parfum : | 0,3% |

### Exemple 18 : Crème hydratante pour peaux grasses

| FORMULE | |
|---|---|
| MONTANOV™ 68 : | 5% |
| Cétylstéaryloctanoate : | 8% |
| Octyl palmitate : | 2% |
| Eau : | q.s.p.100% |
| Composition 1: | 0,6% |
| MICROPEARL™ M100: | 3,0% |
| Mucopolysaccharides: | 5% |
| SEPICIDE™ HB: | 0,8% |
| Parfum: | 0,3% |

### Exemple 19 : Baume après-rasage apaisant sans alcool

| FORMULE | |
|---|---|
| Mélange de lauryl aminoacides | 0,1% à 5% |
| Aspartate de magnésium et de potassium : | 0,002% à 0,5% |
| LANOL™ 99 : | 2% |
| Huile d'amandes douces : | 0,5% |
| Eau : | q.s.p.100% |
| Composition 1 : | 3% |
| SEPICIDE™ HB : | 0,3% |
| SEPICIDE™ CI : | 0,2% |
| Parfum : | 0,4% |

### Exemple 20 : Crème aux AHA pour peaux sensibles

| FORMULE | |
|---|---|
| Mélange de lauryl aminoacides : | 0,1% à 5% |
| Aspartate de magnésium et de potassium : | 0,002% à 0,5% |
| LANOL™ 99 : | 2% |
| MONTANOV™ 68 : | 5,0% |
| Eau : | q.s.p.100% |
| Composition 3 : | 1,50% |
| Acide gluconique: | 1,50% |
| Triéthanolamine : | 0,9% |
| SEPICIDE™ HB : | 0,3% |
| SEPICIDE™ CI : | 0,2% |
| Parfum : | 0,4% |

### Exemple 21 : Soin apaisant après-soleil

| FORMULE | |
|---|---|
| Mélange de lauryl aminoacides: | 0,1% à 5% |
| Aspartate de magnésium et de potassium: | 0,002% à 0,5% |
| LANOL™ 99: | 10,0% |
| Eau: | q.s.p.100% |
| Composition 2: | 2,50% |
| SEPICIDE™ HB: | 0,3% |
| SEPICIDE™ CI: | 0,2% |
| Parfum: | 0,4% |
| Colorant: | 0,03% |

### Exemple 22 : Lait démaquillant

| FORMULE | |
|---|---|
| SEPIPERL™N : | 3% |
| PRIMOL™ 352 : | 8,0% |
| Huile d'amandes douces : | 2% |
| Eau: | q.s.p.100% |
| Composition 1 : | 0,8% |
| Conservateur : | 0,2% |

### Exemple 23 : Lait corporel

| FORMULE | |
|---|---|
| SEPIPERL™ N : | 3,5% |
| LANOL™ 37T : | 8,0% |
| SOLAGUM™ L : | 0,05% |
| Eau: | q.s.p.100% |
| Benzophénone: | 2,0% |
| Diméthicone 350cPs : | 0,05% |
| Poudre de l'exemple 4 : | 0,8% |
| Conservateur : | 0,2% |
| Parfum : | 0,4% |

### Exemple 24 : émulsion fluide à pH alcalin

| | |
|---|---|
| MARCOL™ 82 : | 5,0% |
| NaOH : | 10,0% |
| Eau : | q.s.p.100% |
| Composition 1 : | 1,5% |

### Exemple 25 : Fond de teint fluide

| FORMULE | |
|---|---|
| SIMULSOL™ 165 : | 5,0% |
| LANOL™ 84D : | 8,0% |
| LANOL™ 99 : | 5,0% |
| Eau : | q.s.p.100% |
| Pigments et charges minérales : | 10,0% |
| Composition 3 : | 1,2% |
| Conservateur: | 0,2% |
| Parfum: | 0,4% |

### Exemple 26 : Lait solaire

| FORMULE | |
|---|---|
| SEPIPERL™N : | 3,5% |
| LANOL™ 37T : | 10,0% |
| PARSOL™ NOX: | 5,0% |
| EUSOLEX™ 4360 : | 2,0% |
| Eau: | q.s.p. 100% |
| Composition 1 : | 1,8% |
| Conservateur : | 0,2% |
| Parfum : | 0,4% |

### Exemple 27 : Gel contour des yeux

| FORMULE | |
|---|---|
| Composition 2 : | 2,0% |
| Parfum : | 0,06% |
| Sodium pyrrolidinonecarboxylate : | 0,2% |
| DOW CORNING™ 245 Fluid : | 2,0% |
| Eau : | q.s.p. 100% |

### Exemple 28 : Composition de soin non rincée

| FORMULE | |
|---|---|
| Composition 1 : | 1,5% |
| Parfum : | q.s |
| Conservateur : | q.s. |
| DOW CORNING™ X2 8360 : | 5,0% |
| DOW CORNING™ Q2 1401 : | 15% |
| Eau : | q.s.p. 100% |

### Exemple 29: gel amincissant

| | |
|---|---|
| Composition 3 : | 5% |
| Ethanol : | 30% |
| Menthol : | 0,1% |
| Caféine : | 2,5% |
| Extrait de ruscus : | 2% |
| Extrait de lierre : | 2% |
| SEPICIDE™ HB : | 1% |
| Eau | q.s.p. 100% |

### Exemple 30 : Baume après-rasage apaisant sans alcool

| FORMULE | | |
|---|---|---|
| A | LIPACIDE™ PVB : | 1,0% |
| | LANOL™ 99 : | 2,0% |
| | Huile d'amandes douces : | 0,5% |
| B | Composition 1 : | 3,5% |
| C | Eau : | q.s.p. 100% |
| D | Parfum : | 0,4% |
| | SEPICIDE™ HB : | 0,4% |
| | SEPICIDE™ CI : | 0,2% |

### Exemple 31 : Gel rafraîchissant après-rasage

| FORMULE | | |
|---|---|---|
| A | LIPACIDE™ PVB : | 0,5% |
| | LANOL™ 99 : | 5,0% |
| | Composition 1 : | 2,5% |
| B | eau : | q.s.p.100% |
| C | MICROPEARL™ LM : | 0,5% |
| | Parfum : | 0,2% |
| | SEPICIDE™ HB : | 0,3% |
| | SEPICIDE™ CI : | 0,2% |

### Exemple 32 : Soin pour les peaux grasses

| FORMULE | | |
|---|---|---|
| A | MICROPEARL™ M310 : | 1,0% |
| | Composition 1 : | 5,0% |
| | Isononanoate d'octyle : | 4.0% |
| B | Eau : | q.s.p.100% |
| C | SEPICONTROL™ A5 : | 4,0% |
| | Parfum : | 0,1 % |
| | SEPICIDE™ HB : | 0,3% |
| | SEPICIDE™ CI : | 0,2% |
| D | CAPIGEL™ 98: | 0,5% |
| | Eau : | 10% |

### Exemple 33 : Crème aux AHA

| FORMULE | | |
|---|---|---|
| A | MONTANOV™ 68 : | 5,0% |
| | LIPACIDE™ PVB: | 1,05% |
| | LANOL™ 99 : | 10,0% |
| B | Eau : | q.s.p. 100% |
| | Acide gluconique : | 1,5% |
| | TEA (triéthanolamine) : | 0,9% |
| C | Composition 1 : | 1,5% |
| D | Parfum: | 0,4% |
| | SEPICIDE™ HB: | 0,2% |
| | SEPICIDE™ CI: | 0,4% |

### Exemple 34 : Autobronzant non gras pour visage et corps

| FORMULE | | |
|---|---|---|
| A | LANOL™ 2681 : | 3,0% |
| | Composition 3 : | 2,5% |
| B | Eau: | q.s.p.100% |
| | Dihydroxyacétone : | 3,0% |
| C | Parfum: | 0,2% |
| | SEPICIDE™ HB : | 0,8% |
| | NaOH (hydroxyde de sodium) : | qs pH = 5% |

### Exemple 35 : Lait solaire au monoï de Tahiti

| FORMULE | | |
|---|---|---|
| A | Monoï de Tahiti : | 10% |
| | LIPACIDE™ PVB : | 0,5% |
| | Composition 2 : | 2,2% |
| B | Eau : | q.s.p. 100% |
| C | Parfum: | 0,1% |
| | SEPICIDE™ HB : | 0,3% |
| | SEPICIDE™ CI : | 0,1% |
| | Méthoxycinnamate d'octyle : | 4,0% |

### Exemple 36 : Soin solaire pour le visage

| FORMULE | | |
|---|---|---|
| A | Cyclométhicone et diméthiconol : | 4,0% |
| | Composition 1 | 3,5% |
| B | Eau : | q.s.p. 100% |
| C | Parfum: | 0,1 % |
| | SEPICIDE™ HB : | 0,3% |
| | SEPICIDE™ CI : | 0,21% |
| | Méthoxycinnamate d'octyle : | 5,0% |
| | Micatitane : | 2,0% |
| | Acide lactique : | q.s.p. pH = 6,5 |

### Exemple 37 : Emulsion bronzante sans soleil

| FORMULE | | |
|---|---|---|
| A | LANOL™ 99 : | 15% |
| | MONTANOV™ 68 : | 5,0% |
| | Paraméthoxycinnamate d'octyle : | 3,0% |
| B | Eau : | q.s.p. 100% |
| | Dihydroxyacétone : | 5,0% |
| | Phosphate monosodique : | 0,2% |
| C | Poudre de l'exemple 4 : | 0,5% |
| D | Parfum: | 0,3% |
| | SEPICIDE™ HB : | 0,8% |
| | NaOH : | q.s. pH=5. |

### Exemple 38 : Gel brillance

| | |
|---|---|
| Poudre de l'exemple 4 : | 1,5% |
| Silicone volatile : | 25% |
| Monopropylèneglycol : | 25% |
| Eau déminéralisée : | 10% |
| Glycérine: | qsp 100% |

### Exemple 39 : Gel amincissant

| | |
|---|---|
| Composition 1: | 1,5% |
| Isononylisononanoate : | 2% |
| Caféine : | 5% |
| Ethanol : | 40% |
| MICROPEARL™ LM : | 2% |
| Eau déminéralisée : | qsp 100% |
| Conservateur parfum : | qs |

### Exemple 40 : Lait démaquillant

| | |
|---|---|
| SIMULSOL™ 165 : | 4% |
| MONTANOV™ 202 : | 1% |
| Caprylate-caprate triglyceride : | 15% |
| PECOSIL™ DCT : | 1% |
| Eau déminéralisée | qs |
| CAPIGEL™ 98 | 0,5% |
| Composition 3 : | 1% |
| PROTEOL™ OAT | 2% |
| NaOH : | qsp pH 7 |

### Exemple 41 : Crème solaire

| | |
|---|---|
| SIMULSOL™ 165 | 3% |
| MONTANOV™ 202 | 2% |
| Benzoate C12-C15 | 8% |
| PECOSIL™ PS 100 | 2% |
| Diméthicone | 2% |
| Cyclométhicone | 5% |
| Octyl-méthoxycinnamate | 6% |
| Benzophénone-3 | 4% |
| Oxyde de Titane | 8% |
| Gomme xanthane | 0,2% |
| Butylène-glycol | 5% |
| Eau déminéralisée | qsp 100% |
| Composition 2 : | 1,5% |
| Conservateur, parfum | qs |

### Exemple 42 : Gel de soin peaux mixtes

| | |
|---|---|
| Composition 2 : | 4% |
| Squalane végétal | 5% |
| Dimethicone | 1,5% |
| SEPICONTROL™ A5 | 4% |
| Gomme xanthane | 0,3% |
| Eau | qsp 100% |
| Conservateur, Parfum | qs |

### Exemple 43 : Voile parfumé pour le corps

| | |
|---|---|
| Composition 1 : | 1,5% |
| Cyclométhicone : | 5% |
| Parfum : | 2% |
| MICROPEARL™ M100 : | 5% |
| Glycérine : | 5% |
| Eau déminéralisée : | qsp 100% |

### Exemple 44 : Crème vitaminée

| | |
|---|---|
| SIMULSOL™ 165 : | 5% |
| MONTANOV™ 202 : | 1% |
| Caprylic/capric triglycerides : | 20% |
| Palmitate de vitamine A : | 0,2% |
| Acetate de vitamine E : | 1% |
| MICROPEARL™ M305 : | 1,5% |
| Poudre de l'exemple 4 : | 0,7% |
| Eau | qsp 100% |
| Conservateur, parfum | qs |

### Exemple 45 : Soin antistress pour cheveux

| Formule | |
|---|---|
| Phase A | |
| Eau : | QSP 100% |
| Gomme xanthane : | 0,50% |

| Phase B | |
|---|---|
| SEPICAP™ MP: | 3,00% |

| Phase C | |
|---|---|
| Composition 1 : | 4,00% |

| Phase D | |
|---|---|
| Butylène Glycol : | 5,00% |
| LANOL™ 99 : | 5,00% |
| SEPICIDE™ HB : | 0,30% |
| SEPICIDE™ CI : | 0,20% |
| Parfum | 0,20% |
| Mode Opératoire : Disperser la gomme xanthane dans l'eau avec une défloculeuse. Ajouter ensuite SEPICAP™ MP, puis la composition 1 ; la disperser puis ajouter les ingrédients de la phase D. | |

### Exemple 46 : Masque crème restructurant pour cheveux stressés et fragilisés

| Formule | |
|---|---|
| Phase A | |
| MONTANOV™ 82 : | 3,00% |
| LANOL™ P : | 6,00% |
| AMONYL™ DM : | 1,00% |
| Isononanoate d'isostéaryle : | 5,00% |
| Poudre de l'exemple 4 : | 2,50% |

| Phase B | |
|---|---|
| Eau : | QSP 100% |

| Phase C | |
|---|---|
| SEPICAP™ MP : | 3,00% |
| SEPICIDE™ HB : | 0,30% |
| SEPICIDE™ CI : | 0,20% |
| Mode Opératoire : Fondre la phase A à 75°C. Chauffer la phase B à 75°C. Emulsionner A dans B. Vers 40°C introduire les constituants de la phase C. | |

### Exemple 47 : Gel purifiant pour visage

| Formule | |
|---|---|
| Phase A | |
| MONTALINE™ C 40 : | 7,00% |
| Base nacrante 2078 : | 5,00% |
| Composition 1 : | 2,00% |

| Phase B | |
|---|---|
| Eau : | QSP 100% |

### Exemple 48 : Shampoing colorant

| Formule | |
|---|---|
| Phase A | |
| MONTALINE™ C 40 : | 15,00% |
| Cocamphoacétate disodé : | 5,00% |
| Cetrimonium chloride : | 1,00% |
| SEPIPERL™ N : | 3,00% |
| Composition 3 : | 3,00% |

| Phase B | |
|---|---|
| Couleur | QSP |
| Eau | QSP 100% |

### Exemple 49 : Lotion capillaire

| | |
|---|---|
| Butylène glycol : | 3,0% |
| Composition 1 : | 3,0% |
| SIMULSOL™ 1293 : | 3,0% |
| Acide lactique : | qs. pH = 6 |
| SEPICIDE™ HB : | 0,2% |
| SEPICIDE™CI : | 0,3% |
| Parfum : | 0,3% |
| Eau : | qs. 100% |

### Exemple 50 : Shampooing protecteur et relaxant

| | |
|---|---|
| Amonyl™ 675 SB : | 5,0% |
| Sodium lauroyl éther sulfate à 28% : | 35,0% |
| Poudre de l'exemple 4 : | 3,0% |
| SEPICIDE™ HB : | 0,5% |
| SEPICIDE™CI : | 0,3% |
| Hydroxyde de sodium : | q.s. pH = 7,2 |
| Parfum : | 0,3% |
| Colorant (FDC bleu 1/jaune 5) : | q.s. |
| Eau : | qsp. 100% |

### Exemple 51 : Protecteur "leave-on" , Soin antistress pour cheveux

| | |
|---|---|
| KETROL™T : | 0,5% |
| Mélange de cocoyl aminoacides : | 3,0% |
| Butylèneglycol : | 5,0% |
| DC 1501 : | 5,0% |
| Composition 2 : | 4,0% |
| SEPICIDE™ HB: | 0,5% |
| SEPICIDE™CI : | 0,3% |
| Parfum: | 0,3% |
| Eau : | qsp.100 |

Le MONTANOV^{™} 68 (cétéaryl glucoside), est une composition auto-émulsionnable telle que décrite dans WO 92/06778, commercialisée par la société SEPPIC.

Le MONTANOV™ 82 est un agent émulsionnant à base d'alcool cétéarylique et de cocoylglucoside.

Le MONTANOV™ 202, est une composition APG/alcools gras telle que décrite dans WO9 98/47610, commercialisée par la société SEPPIC.

Le MICROPEARL™ LM est un mélange de squalane, de polyméthylméthacrylate et de menthol, commercialisé par la société SEPPIC.

Le MICROPEARL™ M 100 est une poudre ultra fine au toucher très doux et à action matifiante commercialisée par la société MATSUMO

Le MICROPEARL™ SQL est un mélange de micro particules renfermant du squalane qui se libère sous l'action du massage; il est commercialisé par la société MATSUMO.

Le SEPICIDE™ HB, qui est un mélange de phénoxyéthanol, de méthyl paraben, d'éthylparaben, de propylparaben et de butylparaben, est un agent conservateur commercialisé par la société SEPPIC.

Le SEPICIDE™ CI, imidazolidine urée, est un agent conservateur commercialisé par la société SEPPIC.

PEMULEN™ TR est un polymère acrylique commercialisé par GOODRICH.

Le SIMULSOL™ 165 est du stéarate de glycérol auto-émulsionnable commercialisée par la société SEPPIC.

Le LANOL™ 14M et le LANOL™ S sont des facteurs de consistance commercialisés par la société SEPPIC.

Le LANOL™ 84D est du malate de dioctyle commercialisé par la société SEPPIC.

Le LANOL™ 99 est de l'isononyl isononanoate commercialisé par la société SEPPIC.

Le LANOL™ 37T est du triheptanoate de glycérol, commercialisé par la société SEPPIC.

Le LANOL™ P est un additif à effet stabilisant commercialisé par la société SEPPIC.

Le LANOL™ 1688 est un ester émollient à effet non gras commercialisé par la société SEPPIC.

Le LANOL™ 2681 est un mélange caprylate, caprate de coprah, commercialisé par la société SEPPIC.

Le MONTEINE™ CA est un agent hydratant commercialisé par la société SEPPIC.

Le SCHERCEMOL™ OP est un ester émollient à effet non gras.

Le PARSOL™ MCX est de l'octyl paraméthoxycinnamate; commercialisé par la société GIVAUDAN.

Le SEPIPERL^{™} N est un agent nacrant, commercialisé par la société SEPPIC, à base d'un mélange d'alkyl poly glucosides tels que ceux décrits dans WO 95/13863.

Le SOLAGUM™ L est un carraghénane commercialisé par la société SEPPIC.

Le MARCOL™ 82 est une huile de paraffine commercialisée par la société EXXON.

Le PARSOL™ NOX est un filtre solaire commercialisé par la société GIVAUDAN.

L'EUSOLEX™ 4360 est un filtre solaire commercialisé par la société MERCK.

Le DOW CORNING™ 245 Fluid est de la cyclométhicone, commercialisée par la société DOW CORNING.

Le LIPACIDE™ PVB, est un hydrolysat de protéines de blé acylé commercialisé par la société SEPPIC.

Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.

Le CAPIGEL™ 98 est un copolymère acrylique commercialisé par la société SEPPIC.

Les définitions des produits commerciaux utilisés dans les exemples sont les suivantes:

Le MONTALINE™ C40 : (cocamoniumcarbamoyl chloride) commercialisé par SEPPIC. SEPIPERL™ N : (cocoyl glucoside *1* cocoyl alcohol) commercialisé par SEPPIC. AMONYLT^{M} DM : (Quatemium 82) commercialisé par SEPPIC.

SEPICAP™ MP : (Sodium cocoyl amino acids *1* potassium dimethicone copolyol panthenyl phosphate) commercialisé par SEPPIC.

SIMULSOL™ 1293 est de l'huile de castor hydrogénée et éthoxylée, avec un indice d'éthoxylation égal à 40, commercialisé par la société SEPPIC.

KETROL™ T est de la gomme de xanthane commercialisée par la société KELCO.

DC1501 est un mélange de cyclopentasiloxane et de diméthiconol commercialisé par la société DOW CHEMICAL.

## Revendications

1. Composition comprenant une phase huile, une phase aqueuse, au moins un système émulsionnant de type eau-dans huile, sous forme d'un latex inverse, **caractérisée en ce qu'**elle comprend un polyélectrolyte anionique linéaire, branché ou réticulé, comprenant :
a) une proportion molaire non nulle, d'unités monomériques issues de l'acide acrylique sous forme libre ou partiellement salifié, et
b) une proportion molaire non nulle d'unités monomériques issues de l'acide de formule (I) :
CH₂=CH-C(=O)-O-[CH₂-CH₂-C(=O)-O]ₙ-H (I),
dans laquelle n représente un nombre supérieur ou égal à 1 et inférieur ou égal à 5, ou d'un mélange d'acides de formule (I), sous forme libre ou partiellement salifiée.

2. Composition telle que définie à la revendication 1, dans laquelle ledit polyélectrolyte anionique comprend pour 100% molaire :
- De 90% molaire à 99,5% molaire et plus particulièrement de 95% molaire à 99% molaire d'unités monomériques issues de l'acide acrylique libre ou partiellement salifié ;
- De 0,5% molaire à 10% molaire et plus particulièrement de 1% molaire à 5% d'unités monomériques issues de l'acide carboxylique de formule (I), libre ou partiellement salifié.

3. Composition telle que définie à l'une des revendications 1 ou 2, dans laquelle ledit polyélectrolyte anionique comprend une proportion molaire non nulle d'unité monomériques issues d'un mélange de l'acide de formule (I₁):
CH₂=CH-C(=O)-O-CH₂-CH₂-C(=O)-O-H (I₁),
correspondant à la formule (I) telle que définie précédemment dans laquelle n représente un nombre égal à 1, et de l'acide de formule (I₂) :
CH₂=CH-C(=O)-O-CH₂-CH₂-C(=O)-O-CH₂-CH₂-C(=O)-OH (I₂),
correspondant à la formule (I) telle que définie précédemment dans laquelle n représente un nombre égal à 2, lesdits acides étant sous forme libre ou partiellement salifiée, dans un ratio molaire (I₁)/(I₂) supérieur à 1 et plus particulièrement supérieur à 1,5.

4. Composition telle que définie à la revendication 2, dans laquelle ledit polyélectrolyte anionique comprend une proportion molaire non nulle d'unité monomériques issues d'un mélange de l'acide de formule (I₁):
CH₂=CH-C(=O)-O-CH₂-CH₂-C(=O)-O-H (I₁),
correspondant à la formule (I) telle que définie précédemment dans laquelle n représente un nombre égal à 1, de l'acide de formule (I₂) :
CH₂=CH-C(=O)-O-CH₂-CH₂-C(=O)-O-CH₂-CH₂-C(=O)-OH (I₂),
correspondant à la formule (I) telle que définie précédemment dans laquelle n représente un nombre égal à 2, et de l'acide de formule (I₃) :
CH₂=CH-C(=O)-O-CH₂-CH₂-C(=O)-O-CH₂-CH₂-C(=O)-O-CH₂-CH₂-C(=O)-OH (I₃),
correspondant à la formule (I) telle que définie précédemment dans laquelle n représente un nombre égal à 3, lesdits acides étant sous forme libre ou partiellement salifiée, dans un ratio molaire (I₁)/(I₂) supérieur à 1 et plus particulièrement supérieur à 1,5 et dans un ratio molaire (I₂)/(I₃) supérieur à 1,5 et plus particulièrement supérieur ou égal à 2.

5. Composition telle que définie à l'une quelconque des revendications 1 à 4, dans laquelle le polyélectrolyte anionique tel que défini précédemment est réticulé.

6. Composition telle que définie à l'une quelconque des revendications 1 à 5, comprenant de 15% à 80% massique, dudit polyélectrolyte anionique.

7. Composition telle que définie à l'une quelconque des revendications 1 à 6, comprenant en outre un système émulsionnant de type "huile-dans eau".

8. Procédé de préparation de la composition telle que définie à l'une quelconque des revendications 1 à 7, comprenant les étapes suivantes :
Une étape a) au cours de laquelle une solution aqueuse comprenant les monomères et les éventuels additifs hydrophiles, est émulsionnée dans une phase huile comprenant les monomères et les éventuels additifs lipophiles en présence dudit système émulsifiant de type eau dans huile ;
Une étape b) au cours de laquelle la réaction de polymérisation est amorcée par introduction dans l'émulsion formée à l'issue de l'étape a), d'un initiateur de radicaux libres et d'éventuellement un co-initiateur puis laissée se dérouler pour obtenir ladite composition sous forme d'un latex inverse.

9. Procédé tel que défini à la revendication 8, comprenant en outre une étape c), au cours de laquelle ledit système émulsionnant de type huile dans eau est ajouté au latex-inverse formé à l'issue de l'étape b), pour obtenir ladite composition sous forme d'une latex inverse auto-inversible.

10. Procédé tel que défini à l'une quelconque des revendications 8 ou 9, comprenant en outre une étape b₁, au cours de laquelle le latex inverse issu de l'étape b) est concentré, pour obtenir ladite composition sous forme d'un latex inverse concentré, avant la mise en oeuvre le cas échéant, de l'étape c).

11. Procédé tel que défini à la revendication 10, comprenant en outre une étape c₁, au cours de laquelle le latex inverse auto-inversible issu de l'étape c) est concentré, pour obtenir ladite composition sous forme d'un latex inverse auto-inversible concentré.

12. Procédé tel que défini à l'une quelconque des revendications 8 à 11, comprenant en outre une étape d) au cours de laquelle le latex inverse issu de l'étape b), le latex inverse concentré issu de l'étape b1), le latex inverse auto-inversible issu de l'étape c) ou le latex inverse auto-inversible concentré issu de l'étape c1), est séché par atomisation, pour former une poudre dudit polyélectrolyte anionique.

13. Utilisation de la composition telle que définie à l'une quelconque des revendications 1 à 7, ou de la poudre telle que définie à la revendication 13, comme agent épaississant et/ou comme agent émulsionnant dans des compositions cosmétiques ou pharmaceutiques.

14. Composition cosmétique ou pharmaceutique caractérisée en en ce qu'elle contient comme agent émulsionnant et/ou épaississant, une quantité efficace telle que définie à l'une quelconque des revendications 1 à 7, ou de la poudre obtenue par le procédé tel que défini à la revendication 12.

## Patentansprüche

1. Zusammensetzung, die eine Ölphase, eine wässrige Phase, mindestens ein Emulgatorsystem des Typs Wasser-in-Öl enthält, in Form eines inversen Latex, **dadurch gekennzeichnet, dass** sie einen linearen, verzweigten oder vernetzten anionischen Polymerelektrolyten enthält, enthaltend:
a) einen Molanteil ungleich null von Monomereinheiten, die hervorgegangen sind aus Acrylsäure, die in freier oder teilweise in Salz überführter Form vorliegt, und
b) einen Molanteil ungleich null von Monomereinheiten, die hervorgegangen sind aus der Säure der Formel (I):
CH₂=CH-C(=O)-O-[CH₂-CH₂-C(=O)-O]ₙ-H (I),
worin n eine Zahl größer oder gleich 1 und kleiner oder gleich 5 oder ein Gemisch aus Säuren der Formel (I), die in freier oder teilweise in Salz überführter Form vorliegen, darstellt.

2. Zusammensetzung nach Anspruch 1, in der der anionische Polymerelektrolyt für 100 Mol-% Folgendes enthält;
- 90 Mol-% bis 99,5 Mol-% und insbesondere 95 Mol-% bis 99 Mol-% von Monomereinheiten, die aus freier oder teilweise in ein Salz überführter Acrylsäure hervorgegangen sind;
- 0,5 Mol-% bis 10 Mol-% und insbesondere 1 Mol-% bis 5 Mol-% von Monomereinheiten, die aus freier oder teilweise in ein Salz überführter Carboxylsäure der Formel (I) hervorgegangen sind.

3. Zusammensetzung nach einem der Ansprüche 1 oder 2, in der der anionische Polymerelektrolyt einen Molanteil ungleich null von Monomereinheiten enthält, die hervorgegangen sind aus einem Gemisch der Säure der Formel (I₁):
CH₂=CH-C(=O)-O-CH₂-CH₂-C(=O)-O-H (I₁),
die der oben definierten Formel (I) entspricht, in der n eine ganze Zahl gleich 1 darstellt, und der Säure der Formel (I₂):
CH₂=CH-C(=O)-O-CH₂-CH₂-C(=O)-O-CH₂-CH₂-C(=O)-OH (I₂),
die der oben definierten Formel (I) entspricht, in der n eine ganze Zahl gleich 2 darstellt, wobei die Säuren in freier oder teilweise in Salz überführter Form vorliegen, in einem Molverhältnis (I₁)/(I₂), das größer ist als 1 und insbesondere größer als 1,5.

4. Zusammensetzung nach Anspruch 2, in der der anionische Polymerelektrolyt einen Molanteil ungleich null von Monomereinheiten enthält, die hervorgegangen sind aus einem Gemisch der Säure der Formel (I₁):
CH₂=CH-C(=O)-O-CH₂-CH₂-C(=O)-O-H (I₁),
die der oben definierten Formel (I) entspricht, in der n eine ganze Zahl gleich 1 darstellt, der Säure der Formel (I₂):
CH₂=CH-C(=O)-O-CH₂-CH₂-C(=O)-O-CH₂-CH₂-C(=O)-OH (I₂),
die der oben definierten Formel (I) entspricht, in der n eine ganze Zahl gleich 2 darstellt, und der Säure der Formel (I₃):
CH₂=CH-C(=O)-O-CH₂-CH₂-C(=O)-O-CH₂-CH₂-C(=O)-O-CH₂-CH₂-C(=O)-OH (I₃),
die der oben definierten Formel (I) entspricht, in der n eine ganze Zahl gleich 3 darstellt, wobei die Säuren in freier oder teilweise in Salz überführter Form vorliegen, in einem Molverhältnis (I₁)/(I₂), das größer ist als 1 und insbesondere größer als 1,5, und in einem Molverhältnis (I₂)/(I₃), das größer ist als 1,5 und insbesondere größer oder gleich 2.

5. Zusammensetzung nach einem der Ansprüche 1 bis 4, in der der oben definierte anionische Polymerelektrolyt vernetzt ist.

6. Zusammensetzung nach einem der Ansprüche 1 bis 5, die 15 Masse-% bis 80 Masse-% des anionischen Polymerelektrolyten enthält.

7. Zusammensetzung nach einem der Ansprüche 1 bis 6, die ferner ein Emulgatorsystem des Typs "Öl-in-Wasser" enthält.

8. Verfahren zur Herstellung der Zusammensetzung nach einem der Ansprüche 1 bis 7, umfassend die folgenden Schritte:
einen Schritt a), im dessen Verlauf eine wässrige Lösung, welche die Monomere und die etwaigen hydrophilen Zusatzstoffe enthält, in einer Ölphase, welche die Monomere und die etwaigen lipophilen Zusatzstoffe enthält, in Gegenwart des Emulgatorsystems des Typs Wasser-in-Öl emulgiert wird;
einen Schritt b), in dessen Verlauf die Polymerisationsreaktion durch Einbringen eines Initiators freier Radikaler in die am Ende von Schritt a) gebildete Emulsion und gegebenenfalls eines Co-Initiators eingeleitet wird und anschließend in Entwicklung belassen wird, um die Zusammensetzung in Form eines inversen Latex zu erhalten.

9. Verfahren nach Anspruch 8, das ferner einen Schritt c) umfasst, in dessen Verlauf das Emulgatorsystem des Typs Öl-in-Wasser dem am Ende von Schritt b) gebildeten inversen Latex zugegeben wird, um die Zusammensetzung in Form eines inversen, auto-inversiblen Latex zu erhalten.

10. Verfahren nach einem der Ansprüche 8 oder 9, das ferner einen Schritt b₁ umfasst, im Laufe dessen das aus Schritt b) hervorgegangene inverse Latex konzentriert wird, um die Zusammensetzung in Form eines konzentrierten inversen Latex zu erhalten, bevor gegebenenfalls Schritt c) durchgeführt wird.

11. Verfahren nach Anspruch 10, das ferner einen Schritt c₁ umfasst, in dessen Verlauf das aus Schritt c) hervorgegangene inverse, auto-inversible Latex konzentriert wird, um die Zusammensetzung in Form eines konzentrierten inversen, auto-inversiblen Latex zu erhalten.

12. Verfahren nach einem der Ansprüche 8 bis 11, das ferner einen Schritt d) umfasst, in dessen Verlauf das aus Schritt b) hervorgegangene inverse Latex, das aus Schritt b1) hervorgegangene konzentrierte inverse Latex, das aus Schritt c) hervorgegangene inverse, auto-inversible Latex oder das aus Schritt c1) hervorgegangene konzentrierte inverse, auto-inversible Latex durch Atomisierung getrocknet wird, um ein Pulver des anionischen Polymerelektrolyt zu erhalten.

13. Verwendung der Zusammensetzung nach einem der Ansprüche 1 bis 7 oder des Pulvers nach Anspruch 13 als Verdickungsmittel und/oder als Emulgator in kosmetischen oder pharmazeutischen Zusammensetzungen.

14. Kosmetische oder pharmazeutische Zusammensetzung, **dadurch gekennzeichnet, dass** sie als Verdickungsmittel und/oder Emulgator eine wirksame Menge nach einem der Ansprüche 1 bis 7 oder das Pulver, das durch das Verfahren nach Anspruch 12 erhalten wird, enthält.

## Claims

1. Composition comprising an oil phase, an aqueous phase and at least one water-in-oil type emulsifying system, in the form of an inverse latex, **characterised in that** it comprises a linear, branched or crosslinked anionic polyelectrolyte, comprising:
a) a non-zero molar proportion of monomeric units resulting from acrylic acid in free or partially salified form, and
b) a non-zero molar proportion of monomeric units resulting from the acid of formula (I):
CH₂=CH-C(=O)-O-[CH₂-CH₂-C(=O)-O]ₙ-H (I),
wherein n represents a number greater than or equal to 1 and less than or equal to 5, or from a mixture of acids of formula (I), in free or partially salified form.

2. Composition according to claim 1, wherein said anionic polyelectrolyte comprises, for 100 mol.%:
- from 90 mol.% to 99.5 mol.% and, more particularly, from 95 mol.% to 99 mol.% of monomeric units resulting from the free or partially salified acrylic acid;
- from 0.5 mol.% to 10 mol.% and, more particularly, from 1 mol.% to 5 mol.% of monomeric units resulting from the carboxylic acid of formula (I), which is free or partially salified.

3. Composition according to either claim 1 or claim 2, wherein said anionic polyelectrolyte comprises a non-zero molar proportion of monomeric units resulting from a mixture of the acid of formula (I₁):
CH₂=CH-C(=O)-O-CH₂-CH₂-C(=O)-O-H (I₁)
corresponding to formula (I) as previously defined, wherein n represents a number equal to 1, and of the acid of formula (I₂):
CH₂=CH-C(=O)-O-CH₂-CH₂-C(=O)-O-CH₂-CH₂-C(=O)-OH (I₂),
corresponding to formula (I) as previously defined, wherein n represents a number equal to 2, said acids being in free or partially salified form, in a (I₁)/(I₂) molar ratio greater than 1 and, more particularly, greater than 1.5.

4. Composition according to claim 2, wherein said anionic polyelectrolyte comprises a non-zero molar proportion of monomeric units resulting from a mixture of the acid of formula (I₁):
CH₂=CH-C(=O)-O-CH₂-CH₂-C(=O)-O-H (I₁),
corresponding to formula (I) as previously defined, wherein n represents a number equal to 1, of the acid of formula (I₂):
CH₂=CH-C(=O)-O-CH₂-CH₂-C(=O)-O-CH₂-CH₂-C(=O)-OH (I₂),
corresponding to formula (I) as previously defined, wherein n represents a number equal to 2, and of the acid of formula (I₃):
CH₂=CH-C(=O)-O-CH₂-CH₂-C(=O)-O-CH₂-CH₂-C(=O)-O-CH₂-CH₂-C(=O)-OH (I₃),
corresponding to formula (I) as previously defined, wherein n represents a number equal to 3, said acids being in free or partially salified form, in a (I₁)/(I₂) molar ratio greater than 1 and, more particularly, greater than 1.5 and in a (I₂)/(I₃) molar ratio greater than 1.5 and, more particularly, greater than or equal to 2.

5. Composition according to any of claims 1 to 4, wherein the anionic polyelectrolyte as previously defined is crosslinked.

6. Composition according to any of claims 1 to 5, comprising from 15 % to 80 % by weight of said anionic polyelectrolyte.

7. Composition according to any of claims 1 to 6, further comprising an emulsifying system of the "oil-in-water" type.

8. Method for preparing the composition according to any of claims 1 to 7, comprising the following steps:
step a), during which an aqueous solution comprising the monomers and the optional hydrophilic additives is emulsified in an oil phase comprising the monomers and the optional lipophilic additives in the presence of said emulsifying system of the water-in-oil type;
step b), during which the polymerisation reaction is initiated by introducing a free-radical initiator and, optionally, a coinitiator into the emulsion formed at the end of step a), and is allowed to continue in order to obtain said composition in the form of an inverse latex.

9. Method according to claim 8, further comprising a step c), during which said emulsifying system of the oil-in-water type is added to the inverse latex formed at the end of step b) in order to obtain said composition in the form of a self-invertible inverse latex.

10. Method according to either claim 8 or claim 9, further comprising a step b₁, during which the inverse latex resulting from step b) is concentrated in order to obtain said composition in the form of a concentrated inverse latex, before the implementation of step c), where necessary.

11. Method according to claim 10, further comprising a step c₁, during which the self-invertible inverse latex resulting from step c) is concentrated in order to obtain said composition in the form of a concentrated self-invertible inverse latex.

12. Method according to any of claims 8 to 11, further comprising a step d), during which the inverse latex resulting from step b), the concentrated inverse latex resulting from step b1), the self-invertible inverse latex resulting from step c) or the concentrated self-invertible inverse latex resulting from step c1) is spray-dried in order to form a powder of said anionic polyelectrolyte.

13. Use of the composition according to any of claims 1 to 7, or of said powder according to claim 13, as a thickening agent and/or as an emulsifying agent in cosmetic or pharmaceutical compositions.

14. Cosmetic or pharmaceutical composition, **characterised in that** it contains, as an emulsifying and/or thickening agent, an effective amount of the composition according to any one of claims 1 to 7, or of the powder obtained by means of the method according to claim 12.
